# EUROPEAN PATENT SPECIFICATION

(11) **EP 2 741 671 B1**
(45) Date of publication and mention of the grant of the patent: **13.01.2016**
(21) Application number: 12773352.5
(22) Date of filing: 09.08.2012
(51) Int. Cl.: A61B 5/20, A61B 10/00, G01G 17/04

(54) **URINE FLOW MEASURING APPARATUS**
URINFLUSSMESSER
APPAREIL DE MESURE DU DÉBIT URINAIRE

(30) Priority: 10.08.2011 GB 201113749
(43) Date of publication of application: 18.06.2014
(73) Proprietor: Newcastle-upon-Tyne Hospitals NHS Foundation, High Heaton Newcastle-upon-Tyne Tyne and Wear NE7 7DN (GB)
(72) Inventor: WHITAKER, Michael, Newcastle-upon-Tyne Tyne and Wear NE7 7EX (GB); DRINNAN, Michael, Alnwick Northumberland NE66 1QY (GB); BRAY, Alison, Newcastle-upon-Tyne Tyne and Wear NE13 7JN (GB); GRIFFITHS, Clive, Newcastle-upon-Tyne Tyne and Wear NE7 7RA (GB); BECKWITH, Rober, Ashington Northumberland NE63 0HR (GB)
(74) Representative: Elsworth, Dominic Stephen
(86) International application number: PCT/GB2012/051941
(87) International publication number: WO 2013/021207

(56) References cited:
- WO-A1-00/27285
- WO-A1-2009/107012
- JP-A- 2002 186 601
- US-A1- 2010 064 797

## Description

### Field of the Invention

The invention relates to urine flow measuring apparatus and in particular to portable urine flow measuring apparatus that automatically records urine flow data.

### Background of the Invention

Lower urinary tract symptoms (LUTS) are a common problem affecting around 30% of men aged over 50. LUTS include weak urine flow, the need to urinate frequently and the need to get up at night to urinate. These symptoms often have a detrimental effect on the sufferer's quality of life.

A major cause of male LUTS is non-cancerous enlargement of the prostate which results in the prostate obstructing the outlet of the bladder (bladder outlet obstruction, BOO). This condition can be relieved by drugs or by surgery to remove part of the prostate.

A measurement of the maximum, or peak, rate of urine flow is useful as a clinical indicator of urodynamic obstruction. A low maximum urinary flow rate of less than 15ml/s is a key indication of likely obstruction and indicates that drug or surgical treatment may be beneficial.

Uroflowmetry is the test that measures urinary flow rate, it is the most useful non-invasive technique for the objective assessment of LUTS.

The test is currently typically carried out in a clinic using expensive electronic equipment, such as a weight transducer flow meter, into which the patient must urinate. This is normally followed by an ultrasound scan of the bladder to measure the amount of residual urine in the bladder. The equipment measures the peak flow, voided volume and flow trace with considerable precision. According to good practice the patient should perform two or three voids in the clinic, however there is often not sufficient time in the clinic to achieve this.

In addition to measuring the maximum urinary flow rate in the clinic the patient is often required to complete a 'voiding diary' at home for a number of days. Completion of a 'voiding diary' requires the patient to urinate into a jug and to record the volume, time and date of each voiding event. The 'voiding diary' helps the medical practitioner diagnose the cause of the patient's symptoms.

Problems with the current methodology are that a particular patient's urine flow rate is inherently variable according to the time of day, the amount of fluid consumed and even the availability of the nearest toilet. Therefore, despite the precision of readings obtained by expensive clinic equipment, a single or even two or three measurements of flow recorded in hospital are likely to be unrepresentative of the patient's average flow. Completion of the voiding diary is also important in diagnosing the patient's condition, however measuring each void by hand and remembering to record all the results can be burdensome for patients who may have difficultly keeping a coherent record.

Measurement of urine flow rate in a hospital clinic may also be unpleasant for the patient; sometimes they are required to arrive at the clinic with a full bladder, which in itself is a challenging task for a patient suffering from urinary problems. In order to take repeated measurements the patient must typically attend the clinic for a whole morning or afternoon, spending most of the time in the waiting room drinking copiously in order to repeatedly fill his bladder. The nature of the clinic flow test may prevent the recordal of a representative measurement of urinary flow rate. The clinic apparatus also requires specially trained staff to operate it. The unnatural clinic environment can affect the accuracy of the test result obtained.

US2010/0064797 describes a portable urine flow measuring apparatus comprising a container that receives urine and a scale. The scale may be held by the user during voiding of urine into the container. To use the device the user must first switch it on, then void into the container. The scale measures the weight of the container multiple times at given time intervals. After the device is switched on the measurement data may be recorded onto a memory card in the device. The device is then switched off by the user. This device requires interaction with the user in order to record the voiding data, and this could prove stressful for a user when urgent voiding is required. Failure to activate the device to record the data would mean that the voiding diary is not complete.

WO 00/27285, JP 2002-186601 and WO 2009/107012 also disclose urine flow measuring devices.

It would be desirable to provide an improved portable urine flow measurement apparatus that can be used by the patient to produce a representative measurement and record of urinary flow rate.

### Summary of the Invention

One aspect of the invention, which is defined in independent claim 1 and its dependent claims, provides a urine flow measuring apparatus comprising:
a container having an opening for receiving urine;
measuring means for measuring the weight and/or volume of urine received by the container;
memory means for recording the weight and/or volume of urine measured by the measuring means; and
a controller;
and wherein the controller automatically detects a positive change in weight and/or volume of the container measured by the measuring means and instructs the memory means to record the weight and/or volume of the container at pre-determined time intervals until no further change in weight and/or volume is detected.

Advantageously, the controller automatically detects and/or records only valid positive changes in weight and/or volume.

The controller may automatically detect and/or record positive changes in weight and/or volume above a threshold magnitude.

The controller may include a low pass filter. A low pass filter reduces noise and high frequency changes which may occur if the container is knocked.

The controller may automatically detect and/or record all positive changes in weight and/or volume, with invalid records being removed in a post processing step. The controller may automatically detect and/or record all positive changes in weight and/or volume above a threshold magnitude. The post processing step may include the step of applying a low pass filter to the recorded data.

The controller continually measures the weight and/or volume of the container at regular sampling time intervals. At each sampling time interval the controller identifies if a positive change in the weight and/or volume of the container has occurred and then compares the results from a data set comprising the result from the current sample and results from all the sampling intervals during a given period immediately prior to the current sample. If the number of sampling intervals which indicate a positive change in the weight and/or volume of the container is above a certain threshold value the controller instructs the memory means to begin recording of data. Preferably the controller further instructs the memory means the record the data from the sampling intervals during the given period immediately prior to the current sample. Preferably, if the number of sampling events which indicate a positive change in the weight and/or volume of the container is below a certain threshold value the controller instructs the memory means to cease recording of data.

Preferably the controller continues to measure the weight and/or volume of the container at regular sampling time intervals even when recording of data has ceased.

By comparing data results from a window of activity the apparatus is able to detect and record real urine flow results, and to reject and therefore not record transient apparent changes in weight caused, for example, by knocking the container.

The sampling time intervals are preferably at most one second apart. More preferably, the sampling time intervals are at most 0.25 seconds apart. Still more preferably, the sampling time intervals are at most 0.125 seconds apart.

Preferably the apparatus draws less power when the memory means is not recording data.

Preferably the measuring means measures the weight of urine received by the container and the controller calculates the volume of urine received by the container. The weight of urine received by the container is proportional to the volume of urine received the container. Preferably the controller is calibrated to calculate the volume of the urine and the volume of urine is recorded by the memory means.

The controller may be a micro-processor.

Preferably the memory means automatically records the duration of each urination event and the time between urination events. Preferably, the controller automatically instructs the memory means to record the relative time associated with each change in weight and/or volume of the container.

The memory means for recording the weight and/or volume of urine measured by the measuring means may include a memory chip or a removable memory card.

Weight and/or volume data recorded on the memory means may be transferred to a computer. Computer software may then be used to calculate the rate of change of volume, or the urine flow rate.

The data may be transferred to a computer from the measuring means via USB connection or via Bluetooth or via memory card reader.

Preferably the controller instructs the memory means to record the weight and/or volume of the container at least once every second after a positive change in weight and/or volume has been detected. More preferably, the controller instructs the memory means to record the weight and/or volume of the container at least four times every second. Still more preferably, the controller instructs the memory means to record the weight and/or volume of the container at least eight times every second.

Preferably the apparatus further comprises means for securely engaging the container with the measuring means. This prevents the container from moving or tipping during use.

The means for securely engaging the container with the measuring means may comprise a recess in the base of the container, the recess being shaped to correspond with the shape of the measuring means.

Preferably the opening of the container is funnel shaped. This makes the direction of urine into the container easier for the user.

The urine flow measuring apparatus of the invention provides a convenient, easy to use and reliable means for collecting urine flow measurements from patients in the privacy of their own home.

### Brief Description of the Drawings

In the drawings, which illustrate preferred embodiments of the invention by way of example:
Figure 1 illustrates a urine flow measuring apparatus according to an embodiment of the invention;
Figure 2 illustrates the base unit of the urine flow measuring apparatus of Figure 1, viewed from above;
Figure 3 illustrates an underneath view of the base unit of Figure 2;
Figure 4 illustrates a side view of the apparatus of Figure 1.

### Detailed Description of the Preferred Embodiments

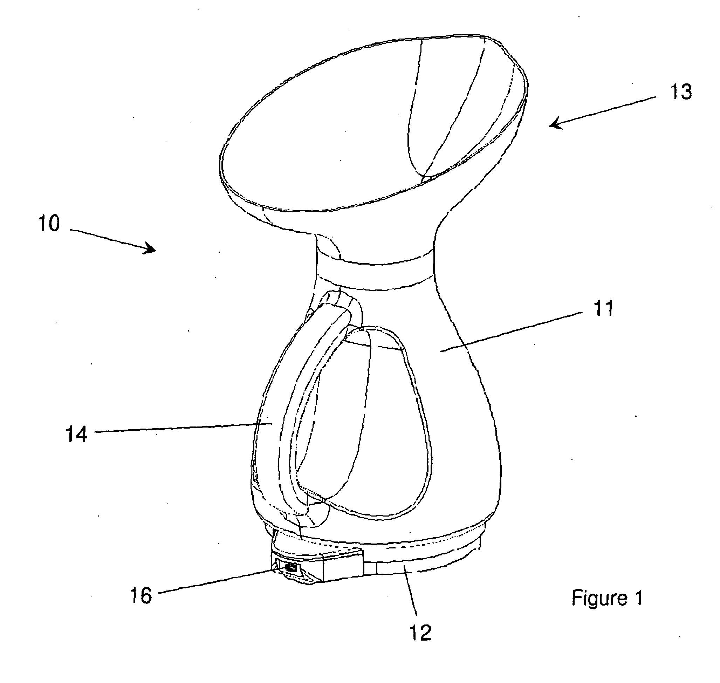

A urine flow measuring apparatus according to an embodiment of the invention is shown generally at 10 in Figure 1. The apparatus 1 includes a jug shaped container 11 that receives urine and a base unit 12 that includes measuring means for measuring the weight and/or volume of the urine received by the container 11. To use the apparatus the container 11 is placed on top of the base unit 12. Preferably the container 11 has a funnel shaped opening portion 13 through which urine voided by an individual is received. The funnel shaped opening portion 13 makes it easier for a user to direct the urine into the container 11. The container 11 is preferably made from plastic, but could be made from any material suitable for containing urine. The container 11 is preferably provided with a handle portion 14. The handle portion 14 makes it easier for the user to transport the container 11 for disposal of the urine and subsequent cleaning.

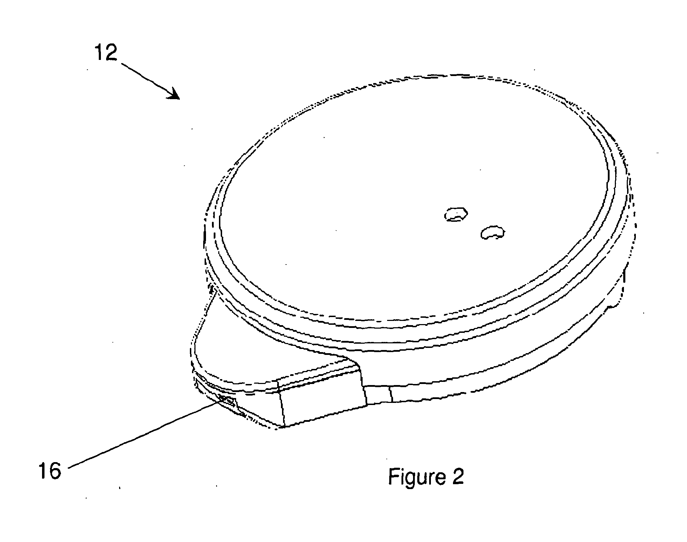

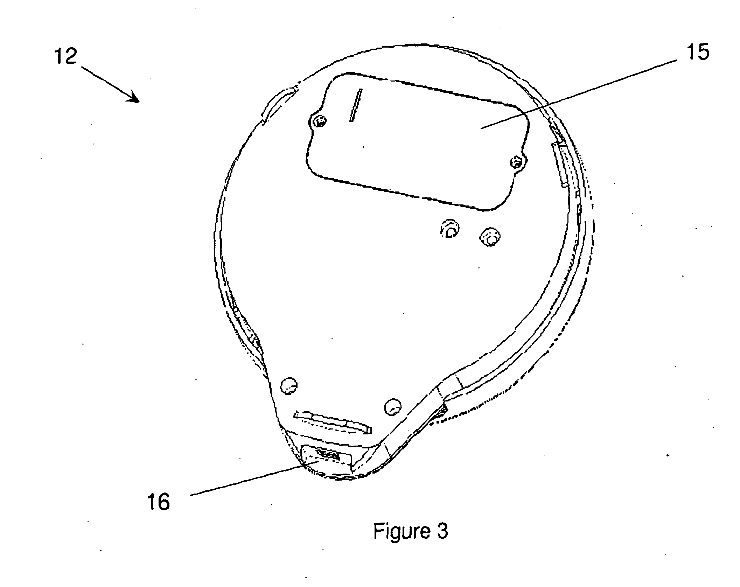

Figure 2 illustrates the base unit 12 in more detail. Preferably the base unit 12 is powered by batteries located inside the battery compartment 15 on the underside of the base unit 12 as shown in Figure 3, but could be powered by another suitable source of electrical energy.

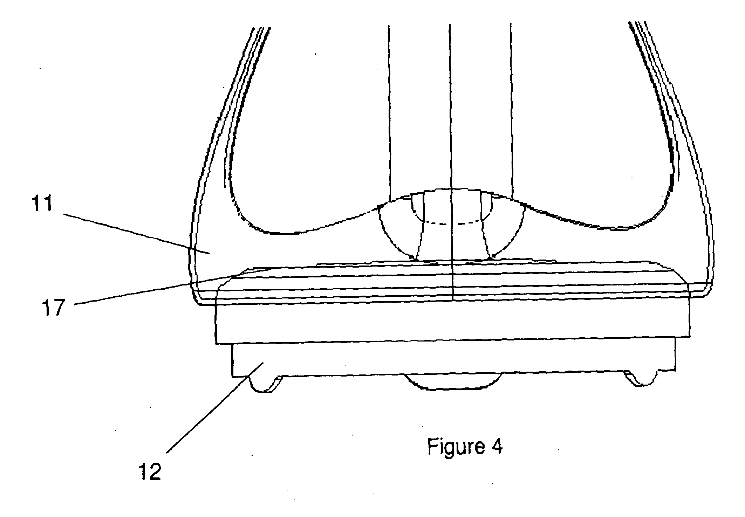

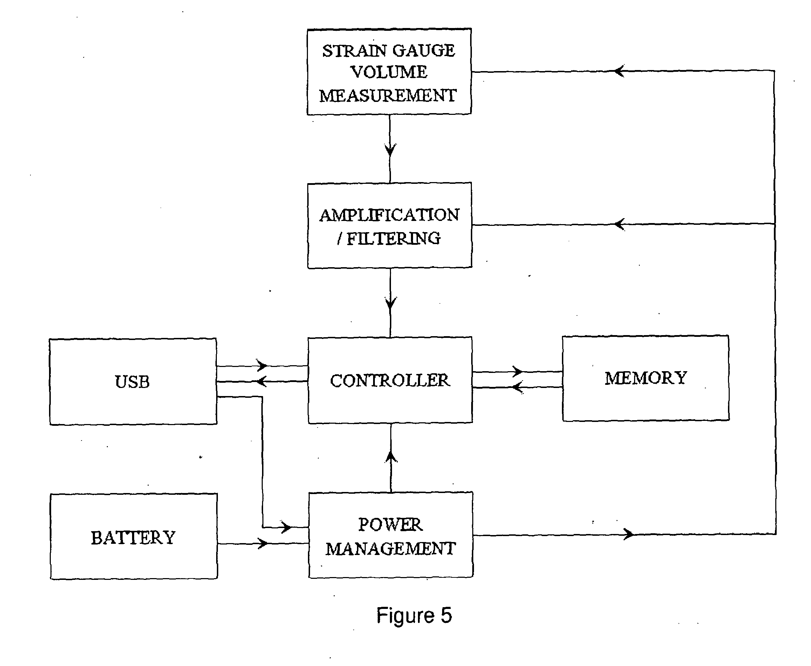

Preferably, as shown in the illustrated embodiment in Figure 4, the base of the jug container 11 has a recessed portion 17 shaped such that it fits over the base unit 12. This means that the container 11 and the base unit 12 fit together securely making it less likely that the jug will move or tip whilst it is being used. As shown in the drawing the edge of the container 11 overhangs the base unit 12, protecting the base unit 12 from any splashes that may occur during use.

The apparatus is used with the jug container 11 positioned on top of the base unit 12. The base unit 12 includes an electronic component called a load beam. As the weight applied to the base unit 12 changes the resistance of the load beam changes. A wheatstone bridge converts the change in resistance to a change in voltage, which changes proportionally to the volume of voided urine received by the container. A controller is located inside the base unit 12. In this example the controller is calibrated to convert the voltage measured to a volume measurement and volume measurements are recorded onto memory located inside the base unit 12.

The controller periodically checks for changes in the weight of the container 11. When a positive change in weight is detected the controller automatically instructs the recording of volume data to the memory chip. Volume data is recorded at pre-determined time intervals until no further change in weight is detected, indicating that voiding has finished. In this example the volume of urine is recorded every 0.125 seconds, or eight times every second. The apparatus is calibrated to take into account the weight of the container 11 when calculating the volume of urine received.

In a preferred embodiment the controller continually measures the weight or volume of the container 11 at pre-determined sampling time intervals. The result from the current sample is then compared with the results of samples taken during a period of time immediately preceding the current sample. For example the weight and/or volume of the container 11 may be measured every 0.125 seconds, and the results compared with the results from sampling events measured during the previous 5 seconds, giving a data set of approximately 40 individual results. In this embodiment of the invention each sample is identified as to whether it relates to a positive change in weight and/or volume of the container 11. If the number of sampling intervals within the data set which indicate a positive change in the weight and/or volume of the container 11 is above a certain threshold value the controller instructs the memory chip to begin recording of data. Preferably the 5 seconds of data from the data set immediately preceding the current sample is also recorded to the memory chip. If the number of sampling events indicating a positive change in the weight and/or volume of the container is below a certain threshold value the controller instructs the memory chip to cease recording of data.

In the illustrated embodiment an internal memory chip for recording information relating to voiding events is located inside the base unit 12. To retrieve the information from the chip the base unit 12 is plugged into a computer via a USB port 16 on the base unit 12 and the data is then downloaded to the computer. The base unit 12 may be brought into the clinic by the patient and the data then downloaded by the clinician. Alternatively, the patient may connect the base unit 12 to their own computer and the data may then be sent to the clinician electronically. Alternatively, a removable memory card may be located inside the base unit 12 for recording information relating to voiding events. The patient may then remove the memory card from the apparatus in order to send it back to the clinician, or may download the data from the memory card onto their own computer and the data may then be sent to the clinician electronically.

Preferably the memory card or the internal memory chip records the date and time (either the actual time or the time relative to the last voiding event) of any voiding event, along with the weight or volume of the voided urine at pre-determined time intervals. The controller ensures that any change in weight of the container 11 is recorded. There is no requirement for the user to switch the apparatus 1 on in order to record the voiding data. When not recording, the apparatus 1 is in a standby mode in which the clock continues to run and the apparatus is ready to record any change in weight of the container.

Once the weight and/or volume and time data have been downloaded to a computer the urine flow rate may be calculated using computer software.

Typically the memory chip of the apparatus can accommodate around 1000 minutes' worth of data which is more than sufficient to record all of an individual's voiding events during a two week period. A two week voiding diary would typically comprise up to 100 minutes of data.

## Claims

1. A urine flow measuring apparatus comprising:
a container having an opening for receiving urine;
measuring means for measuring the weight and/or volume of urine received by the container;
memory means for recording the weight and/or volume of urine measured by the measuring means; and
a controller;
wherein the controller automatically detects a positive change in weight and/or volume of the container and instructs the memory means to record the weight and/or volume of the container at pre-determined time intervals until no further change in weight and/or volume is detected; and
wherein:
the controller continually measures the at least one of weight and volume of the container at regular sampling time intervals; and **characterised in that**:
at each sampling time interval the controller identifies if a positive change in at least one of weight and volume of the container has occurred and then compares the results from a data set comprising the result from the current sample and the results from all the sampling intervals during a given period immediately prior to the current sample; and
if the number of sampling intervals from the data set which indicate a positive change in at least one of weight and volume of the container is above a certain threshold value the controller instructs the memory means to begin recording of data; and
if the number of sampling events from the data set which indicate a positive change in at least one of weight and volume of the container is below a certain threshold value the controller instructs the memory means to cease recording of data.

2. A urine flow measuring apparatus according to Claim 1, wherein the measuring means measures the weight of urine received by the container and the controller calculates the volume of urine received by the container.

3. A urine flow measuring apparatus according to Claim 1 or 2, wherein the controller automatically instructs the memory means to record the relative time associated with each change in weight and/or volume of the container.

4. A urine flow measuring apparatus according to any preceding claim, wherein the memory means includes a memory chip or a removable memory card.

5. A urine flow measuring apparatus according to any preceding claim, wherein the data recorded on the memory means is transferrable to a computer.

6. A urine flow measuring apparatus according to any preceding claim, wherein the pre-determined time interval for recordal of the weight and/or volume of the container is at least once every second.

7. A urine flow measuring apparatus according to any preceding claim, wherein the pre-determined time interval for recordal of the weight and/or volume of the container is at least four times every second.

8. A urine flow measuring apparatus according to any preceding claim, wherein the pre-determined time interval for recordal of the weight and/or volume of the container is at least eight times every second.

9. A urine flow measuring apparatus according to any preceding claim, wherein the controller further instructs the memory means the record the data from the sampling intervals during the given period immediately prior to the current sample.

10. A urine flow measuring apparatus according to any preceding claim, wherein the sampling time intervals are at most one second apart.

11. A urine flow measuring apparatus according to any preceding claim, wherein the sampling time intervals are at most 0.25 seconds apart.

12. A urine flow measuring apparatus according to any preceding claim, wherein the sampling time intervals are at most 0.125 seconds apart.

13. A urine flow measuring apparatus according to any preceding claim, further comprising means for securely engaging the container with the measuring means.

14. A urine flow measuring apparatus according to Claim 13, wherein the container comprises a base and the means for securely engaging the container with the measuring means comprises a recess in the base of the container, the recess being shaped to correspond with the shape of the measuring means.

## Patentansprüche

1. Urinflussmessvorrichtung, die Folgendes umfasst:
einen Container mit einer Öffnung zur Aufnahme von Urin;
Messmittel zum Messen des Gewicht und/oder des Volumens des von dem Container aufgenommenen Urins;
Speichermittel zum Aufzeichnen des Gewichts und/oder des Volumens von von dem Messmittel gemessenem Urin; und
einen Controller;
wobei der Controller automatisch eine positive Änderung des Gewichts und/oder des Volumens des Containers erkennt und das Speichermittel anweist, das Gewicht und/ oder das Volumen des Containers in vorbestimmten Zeitintervallen aufzuzeichnen, bis keine weitere Änderung des Gewichts und/oder des Volumens mehr erkannt wird; und wobei:
der Controller ständig das Gewicht und/oder das Volumen des Containers in regelmäßigen Abtastzeitintervallen misst; und **dadurch gekennzeichnet, dass**:
der Controller in jedem Abtastzeitintervall identifiziert, ob eine positive Änderung des Gewichts und/ oder des Volumens des Containers aufgetreten ist, und dann die Ergebnisse von einem Datensatz, der das Ergebnis von der aktuellen Probe enthält, und die Ergebnisse von allen Abtastintervallen während einer gegebenen Periode unmittelbar vor der aktuellen Probe vergleicht; und
der Controller, wenn die Anzahl von Abtastintervallen aus dem Datensatz, die eine positive Änderung des Gewichts und/oder des Volumens des Containers anzeigen, über einem bestimmten Schwellenwert liegt, das Speichermittel zum Beginnen der Aufzeichnung von Daten anweist; und
der Controller, wenn die Anzahl von Abtastereignissen aus dem Datensatz, die eine positive Änderung des Gewichts und/oder des Volumens des Containers anzeigen, unter einem bestimmten Schwellenwert liegt, das Speichermittel zum Stoppen der Aufzeichnung von Daten anweist.

2. Urinflussmessvorrichtung nach Anspruch 1, wobei das Messmittel das Gewicht von von dem Container aufgenommenem Urin misst und der Controller das Volumen des von dem Container aufgenommenen Urins misst.

3. Urinflussmessvorrichtung nach Anspruch 1 oder 2, wobei der Controller automatisch das Speichermittel zum Aufzeichnen der mit jeder Änderung des Gewichts und/oder des Volumens des Containers assoziierten relativen Zeit anweist.

4. Urinflussmessvorrichtung nach einem vorherigen Anspruch, wobei das Speichermittel einen Speicherchip oder eine entfernbare Speicherkarte beinhaltet.

5. Urinflussmessvorrichtung nach einem vorherigen Anspruch, wobei die auf dem Speichermittel aufgezeichneten Daten auf einen Computer übertragen werden können.

6. Urinflussmessvorrichtung nach einem vorherigen Anspruch, wobei das vorbestimmte Zeitintervall zum Aufzeichnen des Gewichts und/oder des Volumens des Containers wenigstens einmal pro Sekunde ist.

7. Urinflussmessvorrichtung nach einem vorherigen Anspruch, wobei das vorbestimmte Zeitintervall zum Aufzeichnen des Gewichts und/oder des Volumens des Behälters wenigstens viermal pro Sekunde ist.

8. Urinflussmessvorrichtung nach einem vorherigen Anspruch, wobei das vorbestimmte Zeitintervall zum Aufzeichnen des Gewichts und/oder des Volumens des Behälters wenigstens achtmal pro Sekunde ist.

9. Urinflussmessvorrichtung nach einem vorherigen Anspruch, wobei der Controller ferner das Speichermittel zum Aufzeichnen der Daten von den Abtastintervallen während der gegebenen Periode unmittelbar vor der aktuellen Probe anweist.

10. Urinflussmessvorrichtung nach einem vorherigen Anspruch, wobei die Abtastzeitintervalle einen Abstand von höchstens einer Sekunde haben.

11. Urinflussmessvorrichtung nach einem vorherigen Anspruch, wobei die Abtastzeitintervalle einen Abstand von höchstens 0,25 Sekunden haben.

12. Urinflussmessvorrichtung nach einem vorherigen Anspruch, wobei die Abtastzeitintervalle einen Abstand von höchstens 0,125 Sekunden haben.

13. Urinflussmessvorrichtung nach einem vorherigen Anspruch, das ferner Mittel zum sicheren Ineingriffbringen des Containers mit dem Messmittel umfasst.

14. Urinflussmessvorrichtung nach Anspruch 13, wobei der Container eine Basis umfasst und das Mittel zum sicheren Ineingriffbringen des Containers mit dem Messmittel eine Aussparung in der Basis des Containers umfasst, wobei die Aussparung so gestaltet ist, dass sie der Form des Messmittels entspricht.

## Revendications

1. Un appareil de mesure du débit urinaire comprenant :
un récipient présentant une ouverture pour recevoir l'urine ;
un moyen de mesure pour mesurer le poids et/ou le volume d'urine reçu par le récipient ;
un moyen de mémorisation pour enregistrer le poids et/ou le volume d'urine mesuré par le moyen de mesure ; et
un élément de commande ;
dans lequel l'élément de commande détecte automatiquement un changement positif dans le poids et/ou le volume du récipient et donne l'instruction au moyen de mémorisation d'enregistrer le poids et/ou le volume du récipient à des intervalles de temps prédéterminés jusqu'à ce qu'aucun autre changement dans le poids et/ou le volume ne soit détecté ; et dans lequel :
l'élément de commande mesure en continu au moins soit le poids soit le volume du récipient à intervalles de temps d'échantillonnage réguliers ; et **caractérisé en ce que** :
à chaque intervalle de temps d'échantillonnage l'élément de commande identifie si un changement positif d'au moins soit le poids soit le volume du récipient a eu lieu, puis compare les résultats d'un ensemble de données comprenant le résultat de l'échantillon en cours et les résultats de tous les intervalles d'échantillonnage durant une période donnée immédiatement avant l'échantillon en cours ; et
si le nombre d'intervalles d'échantillonnage provenant de l'ensemble de données qui indique un changement positif d'au moins soit le poids soit le volume du récipient est au-dessus d'une certaine valeur de seuil l'élément de commande donne l'instruction au moyen de mémorisation de commencer l'enregistrement des données ; et
si le nombre d'événements d'échantillonnage provenant de l'ensemble de données qui indique un changement positif d'au moins soit le poids soit le volume du récipient est au-dessous d'une certaine valeur de seuil l'élément de commande donne l'instruction au moyen de mémorisation de cesser l'enregistrement des données.

2. Un appareil de mesure du débit urinaire selon la revendication 1, dans lequel le moyen de mesure mesure le poids d'urine reçu par le récipient et l'élément de commande calcule le volume d'urine reçu par le récipient.

3. Un appareil de mesure du débit urinaire selon la revendication 1 ou 2, dans lequel l'élément de commande donne automatiquement l'instruction au moyen de mémorisation d'enregistrer le temps relatif associé à chaque changement en poids et/ou en volume du récipient.

4. Un appareil de mesure du débit urinaire selon l'une quelconque des revendications précédentes, dans lequel le moyen de mémorisation inclut une puce de mémoire ou une carte de mémoire amovible.

5. Un appareil de mesure du débit urinaire selon l'une quelconque des revendications précédentes, dans lequel les données enregistrées sur le moyen de mémorisation sont transférables à un ordinateur.

6. Un appareil de mesure du débit urinaire selon l'une quelconque des revendications précédentes, dans lequel l'intervalle de temps prédéterminé pour l'enregistrement du poids et/ ou du volume du récipient est au moins d'une fois par seconde.

7. Un appareil de mesure du débit urinaire selon l'une quelconque des revendications précédentes, dans lequel l'intervalle de temps prédéterminé pour l'enregistrement du poids et/ ou du volume du récipient est au moins de quatre fois par seconde.

8. Un appareil de mesure du débit urinaire selon l'une quelconque des revendications précédentes, dans lequel l'intervalle de temps prédéterminé pour l'enregistrement du poids et/ ou du volume du récipient est au moins de huit fois par seconde.

9. Un appareil de mesure du débit urinaire selon l'une quelconque des revendications précédentes, dans lequel l'élément de commande donne en outre l'instruction au moyen de mémorisation d'enregistrer les données provenant des intervalles d'échantillonnage durant la période donnée immédiatement avant l'échantillon en cours.

10. Un appareil de mesure du débit urinaire selon l'une quelconque des revendications précédentes, dans lequel les intervalles de temps d'échantillonnage sont au plus d'une seconde.

11. Un appareil de mesure du débit urinaire selon l'une quelconque des revendications précédentes, dans lequel les intervalles de temps d'échantillonnage sont au plus de 0,25 seconde.

12. Un appareil de mesure du débit urinaire selon l'une quelconque des revendications précédentes, dans lequel les intervalles de temps d'échantillonnage sont au plus de 0,125 seconde.

13. Un appareil de mesure du débit urinaire selon l'une quelconque des revendications précédentes, comprenant en outre un moyen pour engager de façon sûre le récipient avec le moyen de mesure.

14. Un appareil de mesure du débit urinaire selon la revendication 13, dans lequel le récipient comprend une base et le moyen pour engager de façon sûre le récipient avec le moyen de mesure comprend un renfoncement dans la base du récipient, le renfoncement étant d'une forme correspondant à la forme du moyen de mesure.
